(19) Europäisches Patentamt
European Patent Office
Office européen des brevets

(11) Veröffentlichungsnummer: **0 221 846**
**A1**

(12) # EUROPÄISCHE PATENTANMELDUNG

(21) Anmeldenummer: 86810474.6

(22) Anmeldetag: 22.10.86

(51) Int. Cl.⁴: C 07 F 7/18
//C07D205/08, C07D499/00

(30) Priorität: 28.10.85 CH 4626/85

(43) Veröffentlichungstag der Anmeldung:
13.05.87 Patentblatt 87/20

(84) Benannte Vertragsstaaten:
AT BE CH DE ES FR GB IT LI LU NL SE

(71) Anmelder: CIBA-GEIGY AG
Klybeckstrasse 141
CH-4002 Basel(CH)

(72) Erfinder: Kalvoda, Jaroslav, Dr.
Leimgrubenweg 21
CH-4102 Binningen(CH)

(72) Erfinder: Ernest, Ivan, Dr.
Rüttihard 10
CH-4127 Birsfelden(CH)

(72) Erfinder: Biollaz, Michel, Dr.
Im Hirshalm 50
CH-4125 Riehen(CH)

(72) Erfinder: Hungerbühler, Ernst, Dr.
Zeisigweg 4
CH-4310 Rheinfelden(CH)

(54) **Optisch aktive Acyloxyazetidinone.**

(57) Optisch aktive Acyloxyazetidinone der Formel

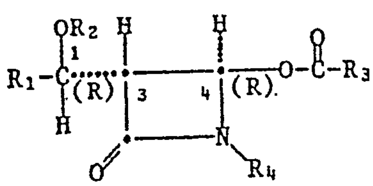

(I),

und Acylazetidinone der Formel

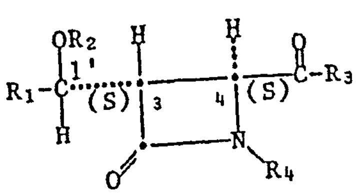

(II),

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R'_4$ bedeuten, und Verfahren zu ihrer Herstellung. Die Verbindungen der Formeln I und II finden Verwendung als Zwischenprodukte in der Penemsynthese.

EP 0 221 846 A1

Croydon Printing Company Ltd.

0221846

CIBA-GEIGY AG
Basel (Schweiz)

4-15580/+

# Optisch aktive Acyloxyazetidinone

Die Erfindung betrifft optisch aktive Acyloxyazetidinone und Verfahren zu Ihrer Herstellung, die als Zwischenprodukte in Verfahren zur Herstellung von antibiotisch wirksamen Penem- oder Carbapenemverbindungen verwendet werden können.

Die Markteinführung und der kommerzielle Erfolg von neuen Heilmitteln ist immer mehr an ein preislich günstiges Verfahren zu ihrer Herstellung gebunden. Die hochaktiven Penem- und Carbapenenantibiotika können nur relativ teuer hergestellt werden, da ihre Synthese über optisch aktive Zwischenprodukte führt und die Anzahl der Verfahrensstufen relativ gross ist. Es besteht daher die Aufgabe, nach Zwischenprodukten zu suchen, die verfahrensmässig die gleichen Vorteile haben wie die bisher bekannten, indem sie beispielsweise in guter Ausbeute, optisch rein, und in für die Lagerung stabilen, z.B. kristallinen Zustand, hergestellt werden können und die darüber hinaus preisgünstig sind. Die vorliegende Erfindung stellt solche Zwischenprodukte in Gestalt der Verbindungen der Formeln I und II zur Verfügung, deren Charakteristikum in der Anwesenheit der Dimethyl-(2,3-dimethylbut-2-yl)silylschutzgruppe $R_2$ und der 4-Acetoxygruppe liegt.

Die Erfindung betrifft in erster Linie optisch aktive Acyloxyazetidinone der Formel

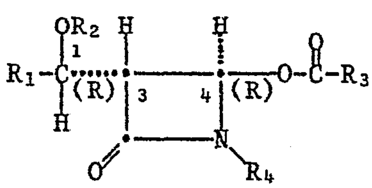

(I) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeuten, und ein Verfahren zu ihrer Herstellung.

Die im Rahmen dieser Erfindung verwendeten allgemeinen Definitionen haben vorzugsweise die folgenden Bedeutungen:

Der im Zusammenhang mit Gruppen oder Resten, z.B. Niederalkyl, Niederalkoxy, Niederalkanoyl etc., verwendete Ausdruck "Nieder" bedeutet, dass die so bezeichneten Gruppen oder Reste, falls nicht ausdrücklich anders definiert, bis einschliesslich 7, bevorzugt bis einschliesslich 4, C-Atome enthalten.

Niederalkyl $R_1$ ist vorzugsweise Methyl, ferner Ethyl, n-Propyl, Isopropyl, n-Butyl, Isobutyl oder tert-Butyl.

$R_1$ ist vorzugsweise Methyl oder auch Wasserstoff.

Eine Aminoschutzgruppe $R_4'$ ist vorzugsweise oxidativ abspaltbares Arylmethyl oder Aryl, wobei Aryl bevorzugt durch eine oder mehrere, z.B. zwei, Niederalkoxygruppen, z.B. Methoxy, substituiertes Phenyl ist, und insbesondere 4-Methoxybenzyl oder 2,4-Dimethoxybenzyl bzw. 4-Methoxyphenyl oder 3,4-Dimethoxyphenyl bedeutet.

Das erfindungsgemässe Verfahren ist dadurch gekennzeichnet, dass man eine Verbindung der Formel

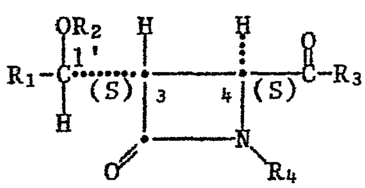

(II) ,

worin $R_1$, $R_2$, $R_3$ und $R_4$ die unter Formel I genannten Bedeutungen haben, durch Behandeln mit einer Persäure einer Baeyer-Villiger-Reaktion unterwirft, oder in einer Verbindung der Formel I, worin $R_2$ Wasserstoff bedeutet, die freie Hydroxygruppe in die geschützte

Hydroxygruppe $OR_2$ überführt, worin $R_2$ Dimethyl-(2,3-dimethylbut-2-yl)silyl ist, und/oder gewünschtenfalls in einer erhältlichen Verbindung der Formel I die Aminoschutzgruppe $R_4'$ abspaltet.

Bevorzugt ist in einem Ausgangsmaterial der Formel II $R_1$ Methyl, $R_4$ Wassserstoff oder eine Arylgruppe, insbesondere p-Methoxyphenyl.

In die Reaktion können organische, anorganische Persäuren oder Iminopercarbonsäuren oder Salze davon, wie Alkalimetall- oder Erdalkalimetallsalze, z.B. Natrium-, Kalium- oder Magnesiumsalze, eingesetzt werden, z.B. gegebenenfalls halogenierte Niederalkanpercarbonsäuren, wie Peressigsäure oder Trifluorperessigsäure, gegebenenfalls durch Nitro und/oder Halogen mono- oder disubstituierte aromatische Persäuren, wie Perbenzoesäure, p-Nitroperbenzoesäure, m-Chlorperbenzoesäure, 3,5-Dinitroperbenzoesäure, Monoperphthalsäure oder ein Salz davon, z.B. Magnesium-monoperphthalat, Peroxoschwefelsäuren, z.B. Peroxoschwefelsäure, Trihalogeniminoperessigsäure, z.B. Trichlor- oder Trifluoriminoporessigsäure. Bevorzugt sind Peressigsäure, m-Chlorperbenzoesäure und Monoperphthalsäure.

Die Persäuren können auch in situ gebildet werden, z.B. aus der entsprechenden Säure oder einem sauren Salz davon und Wasserstoffperoxid. Insbesondere die Iminopercarbonsäuren werden üblicherweise in situ hergestellt, z.B. aus den entsprechenden Nitrilen und Wasserstoffperoxid, bevorzugt in Gegenwart eines schwach sauren Puffers, z.B. Kaliumhydrogenphosphat. Die Reaktion findet in einem üblichen Lösungsmittel, z.B. einem halogenierten Kohlenwasserstoff, wie Methylenchlorid oder Chloroform, einem Ester, wie Essigsäureethylester, einer flüssigen Säure, wie Eisessig, oder einem Alkohol, wie einem niederen Alkanol, z.B. Ethanol, bei Temperaturen zwischen etwa 0° und etwa 100°C, gegebenenfalls in einer Inertgasatmosphäre, und gegebenenfalls unter Druck, z.B. bei etwa bis zu 10 bar, statt.

Die Dimethyl-2,3-dimethylbut-2-ylsilylgruppe kann in an sich bekannter Weise eingeführt werden, z.B. durch Umsetzung einer Verbindung der Formel I, worin $R_2$ Wasserstoff und $R_4$ bevorzugt eine Aminoschutzgruppe $R_4'$ bedeutet, mit einem entsprechenden Halogensilan, z.B. Dimethyl-(2,3-dimethylbut-2-yl)-chlorsilan.

Die Arylmethylgruppe $R_4'$, z.B. 4-Methoxy- oder 2,4-Dimethoxybenzyl, wird z.B. oxidativ abgespalten, z.B. durch Umsetzung mit einem starken Oxydationsmittel, z.B. einem anorganischen Peroxosalz, z.B. Natrium- oder Kaliumperoxodisulfat, in Gegenwart eines sauren Salzes, z.B. Dikaliumhydrogenphosphat, und gegebenenfalls in Gegenwart eines Katalysators, z.B. eines Metallsalzes, z.B. eines Eisen(II)- und/oder Kupfer(II)-salzes, z.B. Eisen(II)-sulfatheptahydrat und/oder Kupfer(II)-acetathydrat, oder insbesondere durch Umsetzung mit einem Cer(IV)-Salz, z.B. Cer(IV)iodat, Cer(IV)-nitrat ($CeOH(NO_3)_3$), Cer(IV)-sulfat oder, bevorzugt mit Diammonium-Cer(IV)-hexanitrat, z.B. in wässrig-organischen Medien, bevorzugt in wässrigem Acetonitril.

Die Arylgruppe $R_4'$, z.B. 4-Methoxyphenyl oder 3,4-Dimethoxyphenyl, wird bevorzugt oxidativ abgespalten, z.B. ozonolytisch in Ethylacetat und anschliessende Zersetzung der gebildeten Ozonide durch Reduktion mit einem Reduktionsmittel, z.B. Natriumthiosulfat, oder durch Umsetzung mit einem Cer(IV)-Salz, z.B. Diammonium-Cer(IV)-hexanitrat.

$R_4'$ kann selektiv, d.h. unter Erhaltung der Schutzgruppe $R_2$ abgespalten werden.

Die beschriebenen Spaltungsreaktionen werden unter an sich bekannten Bedingungen durchgeführt, wenn notwendig unter Kühlen oder Erwärmen und gegebenenfalls in einer Inertgas-, z.B. Stickstoffatmosphäre.

Die vorliegende Erfindung hat vorzugsweise ein Verfahren zur Herstellung von Verbindungen der Formel I zum Gegenstand, worin $R_1$ Methyl, $R_2$ Dimethyl-(2,3-dimethylbut-2-yl)silyl, $R_3$ Methyl und $R_4$

Wasserstoff oder eine Aminoschutzgruppe $R_4^t$, z.B. 4-Methoxybenzyl, 2,4-Dimethoxybenzyl, 4-Methoxyphenyl oder 3,4-Dimethoxyphenyl, bedeuten, und worin das 1'-C-Atom der Seitenkette die R- oder die S-Konfiguration hat.

Verbindungen der Formel I, insbesondere solche, worin $R_1$ Methyl und $R_4$ Wasserstoff ist, sind wertvolle Zwischenprodukte, die auf an sich bekannte Weise zu antibiotisch wirksamen Penem- oder Carbapenemerbindungen weiterverarbeitet werden können. Die Weiterverarbeitung erfolgt analog derjenigen von bekannten Azetidinonverbindungen z.B. wie von T. Hayashi et al., Chem. Pharm. Bull. 29, 3158, 1981, oder in den Europäischen Patentanmeldungen Nr 42026-A (für Peneme) oder Nr. 78026-A (für Carbapeneme) beschrieben ist.

Die Dimethyl-(2,3-dimethylbut-2-yl)silylgruppe $R_2$ in einer Verbindung der Formel I und daraus erhältlichen Folgeprodukten, insbesondere den entsprechenden Penemen, kann in Gegenwart hydroxylgruppenhaltiger Lösungsmittel neutral, sauer oder alkalisch, oder insbesondere durch Behandeln mit einem Fluoridanionen liefernden Salz der Fluorwasserstoffsäure, wie mit einem Alkalimetallfluorid, z.B. Natriumfluorid, gegebenenfalls in Anwesenheit eines makrocyclischen Polyethers ("Kronenether"), oder mit dem Fluorid einer organischen quaternären Base, wie Tetra-niederalkylammoniumfluorid, z.B. Tetrabutylammoniumfluorid, oder insbesondere einer Fluorwasserstoff-Harnstoff-Mischung gegebenenfalls in Gegenwart einer Base, z.B. 2,6-Lutidin abgespalten werden.

Die Erfindung betrifft ebenfalls die Ausgangsverbindungen der Formel II, worin $R_1$, $R_2$, $R_3$ und $R_4$ die unter Formel I genannten Bedeutungen haben, und Verfahren zu ihrer Herstellung.

Das Verfahren zur Herstellung von Verbindungen der Formel II ist dadurch gekennzeichnet, dass man a) ein α-Carbanion einer Verbindung der Formel

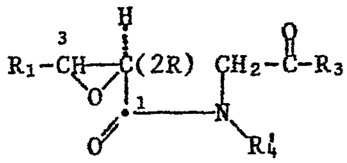

(III) ,

worin $R_1$, $R_3$ und $R'_4$ die unter Formel I genannten Bedeutungen haben, und das 2-C-Atom die R- und, wenn $R_1$ Niederalkyl bedeutet, das 3-C-Atom die R- oder die S-Konfiguration hat, oder eine Verbindung der Formel

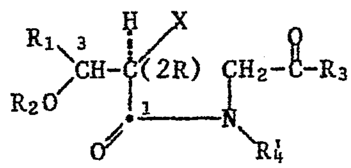

(IV),

worin $R_1$, $R_2$, $R_3$ und $R'_4$ die unter Formel I genannten Bedeutungen haben, und X eine nukleofuge Abgangsgruppe darstellt und das 2-C-Atom die R- und, wenn $R_1$ Niederalkyl bedeutet, das 3-C-Atom die R- oder die S-Konfiguration hat, zyklisiert, oder b) eine cis-Verbindung der Formel

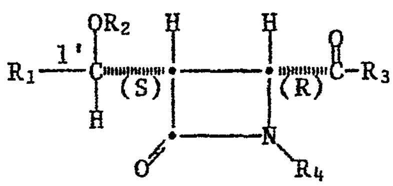

(II') ,

worin $R_1$, $R_2$, $R_3$ und $R_4$ die unter Formel II angegebenen Bedeutungen haben, isomerisiert, und, wenn erwünscht, die Aminoschutzgruppe $R'_4$ abspaltet und durch Wasserstoff ersetzt, und/oder, eine erhältliche Verbindung der Formel II, worin $R_2$ Wasserstoff ist, mit einem Dimethyl-(2,3-dimethylbut-2-yl)-halogensilan umsetzt.

In einem Ausgangsmaterial der Formel IV ist eine geeignete nukleofuge Abgangsgruppe X beispielsweise Halogen, z.B. Chlor, Brom oder Jod, Niederalkanoyloxy, z.B. Acetoxy, Arylsulfonyloxy, z.B. Phenylsulfonyloxy oder Tosyloxy, oder Niederalkylsulfonyloxy, z.B. Mesyloxy.

Unter dem α-Carbanion einer Verbindung der Formeln III oder IV ist ein Anion zu verstehen, bei dem sich die negative Ladung auf dem Kohlenstoffatom in α-Stellung zur $-CO-R_3$ Gruppe befindet, d.h. eine entsprechende Verbindung mit der Teilformel $-N-CH^{\ominus}-CO-R_3$. Solche Carbanionen werden in situ hergestellt, indem man eine Verbindung der Formeln III oder IV in einem geeigneten Lösungsmittel mit einer Base behandelt, die solche Carbanionen bildet. Solche Basen sind beispielsweise Alkalimetallbasen, wie Hydroxide oder Carbonate von Natrium, Kalium oder Lithium, oder organische Alkalimetallverbindungen, z.B. Niederalkyllithiumverbindungen, wie n-Butyl- oder tert-Butyllithium, Alkalimetallamide, z.B. Lithiumdiisopropylamid oder -di-tert-butylamid, und insbesondere Alkalimetallamide, worin zwei Wasserstoffatome durch Silyl-, z.B. Trimethylsilylgruppen ersetzt sind, z.B. Lithium-, Natrium- oder Kalium-bis-(trimethylsilyl)-amid.

Weitere Basen sind organische Stickstoffbasen, z.B. Amidine, wie 1,5-Diazabicyclo[4.3.0]non-5-en und 1,5-Diazabicyclo[5.4.0]undec-5-en, sowie Reagenzien, die in einem aprotischen, organischen Lösungsmittel Fluoridionen liefern und besonders zur Herstellung von Carbanionen der Verbindungen der Formel III geeignet sind.

Ein Reagenz, welches in einem aprotischen, organischen Lösungsmittel Fluoridionen liefert, ist vorzugsweise ein Tetra-niederalkylammoniumfluorid, insbesondere Tetra-n-butylammoniumfluorid.

Geeignete Lösungsmittel zur Carbanionbildung sind beispielsweise Kohlenwasserstoffe, wie Benzol oder Toluol, halogenierte Kohlenwasserstoffe, wie Methylenchlorid oder Chloroform, Ether, wie Dioxan, Tetrahydrofuran oder Dimethoxyethan, Amide, wie Dimethylformamid oder Hexamethylphosphorsäuretriamid, Sulfoxide, wie Dimethylsulfoxid oder Nitrile, wie Acetonitril, oder Mischungen davon. Die Reaktion kann auch unter Phasentransferbedingungen vorgenommen werden, z.B. im System bestehend aus Methylenchlorid und 50 prozentiger Natronlauge in Gegenwart eines Phasentransferkatalysators, wie Benzyltriethylammoniumchlorid oder Tetrabutylammoniumhydrogensulfat. Die Reaktionstemperatur hängt ebenfalls von der verwendeten Base ab und liegt zwischen ca. -80°C und 80°C. Man arbeitet vorzugsweise unter Inertgasatmosphäre, z.B. Argon- oder Stickstoffatmosphäre.

Bei dem Verfahren findet eine Inversion der Konfiguration am 2-C-Atom des Ausgangsmaterials statt, so dass aus einer 2R-Verbindung der Formeln III oder IV eine 3S-Verbindung der Formel II entsteht. Die Konfiguration des 1'-C-Atoms ($R_1$ = Niederalkyl) in der Seitenkette bleibt unverändert. Beispielsweise erhält man aus R-Glycidsäureamiden der Formel (III) 3S-Azetidinone der Formel II oder aus (2 R,3 R)-2,3-Epoxybuttersäureamiden der Formel III (3S-1'R)-3-Hydroxyethylazetidinone der Formel II.

Das Isomerisierungsverfahren b) findet in einem geeigneten Lösungsmittel oder Lösungsmittelgemisch in Gegenwart einer Base statt. Geeignete Lösungsmittel und Basen sowie auch die Reaktionsbedingungen sind die gleichen, die für das Zyklisierungsverfahren a) eingesetzt werden können. Bevorzugt werden aprotische dipolare Lösungsmittel, wie Dimethylformamid, N-Methylpyrrolidin, Dimethylsulfoxid oder dergleichen eingesetzt. Als Basen kommen vor allem Kaliumcarbonat oder 1,5-Diazabicyclo[5.4.0]undec-5-en in Frage, wobei auch unter den genannten Phasentransferbedingungen gearbeitet werden kann.

Die cis-Verbindungen der Formel II' können bei Zyklisierungsreaktion a) als Nebenprodukte entstehen. Sie können auf übliche Weise, z.B. durch Chromatographieren, abgetrennt werden.

Verbindungen der Formel III können in an sich bekannter Weise durch Umsetzung von Verbindungen der Formel VII mit Epoxysäuren der Formel

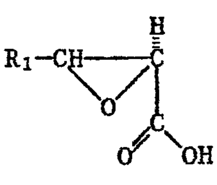

(VIII)

oder deren Salzen hergestellt werden, analog wie weiter unten für die Umsetzung einer Carbonsäure der Formel VI mit einem Amin der Formel VII angegeben ist. Die Epoxysäuren der Formel VIII sind bekannt oder können auf an sich bekannte Weise hergestellt werden.

In einer Ausführungsform des Verfahrens werden Ausgangsmaterialien der Formel III aus Verbindungen der Formel

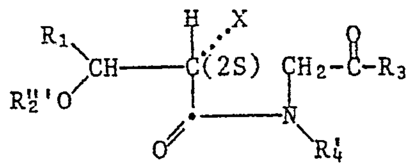

(V),

worin $R_1$, $R_3$, $R_4'$ und X die genannten Bedeutungen haben, $R_2''{}'$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ Niederalkyl bedeutet, unter den genannten carbanionbildenden Bedingungen und unter Waldenscher Umkehr am 2-C-Atom <u>in situ</u> hergestellt.

In einer Verbindung der Formel V ist eine unter den Bedingungen der Epoxidbildung abspaltbare Hydroxyschutzgruppe $R_2''{}'$ eine Triniederalkylsilylgruppe, z.B. Dimethyl-tert-butylsilyl oder Trimethylsilyl. X bedeutet bevorzugt Halogen, z.B. Chlor oder Brom, Niederalkansulfonyloxy, z.B. Mesyloxy, oder Arylsulfonyloxy, z.B. Tosyloxy.

Die Abspaltung von $R_2''{}'$-X, worin $R_2''{}'$ Wasserstoff ist, erfolgt mit einer der genannten carbanionbildenden Basen, z.B. Natrium- oder Kaliumhydroxid oder Natrium- oder Kaliumcarbonat, vorzugsweise mit einem Amidin, z.B. 1,5-Diazabicyclo[5.4.0]undec-5-en, oder auch mit einem Tetraniederalkylammoniumfluorid, z.B. entwässertem Tetra-n-butylammoniumfluorid. Wenn $R_2''{}'$ eine unter den Bedingungen der Epoxidbildung abspaltbare Triniederalkylsilylgruppe ist, wird bevorzugt ein Tetraniederalkylammoniumfluorid verwendet.

Die Umsetzung zu einer Epoxyverbindung der Formel III wird vorzugsweise in einem inerten, vorzugsweise wasserfreien, Lösungsmittel oder Lösungsmittelgemisch vorgenommen, beispielsweise in einem Carbonsäureamid, z.B. Dimethylformamid, einem halogenierten Kohlenwasserstoff, z.B. Methylenchlorid, Chloroform, Tetrachlorkohlenstoff oder Chlorbenzol, einem Keton, z.B. Aceton, einem cyclischen Ether, z.B. Dioxan oder Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Bei der Umsetzung gemäss diesem Verfahren kehrt sich die Konfiguration am 2-C-Atom um, während die Konfiguration des 3-C-Atoms ($R_1$ = Niederalkyl) erhalten bleibt. Aus 2S-Halogen-3R-hydroxycarbonsäureamiden der Formel V entstehen die (2R,3R)-Verbindungen der Formel III. Entsprechendes gilt für die 2S-Halogen-3S-hydroxycarbonsäureamide der Formel V.

Das Epoxid der Formel III kann auch nach seiner Herstellung durch Abspaltung von $R_2''$'-X aus einer Verbindung der Formel V isoliert werden.

Die Umwandlung einer Verbindung der Formel V, worin $R_2''$' Wasserstoff ist, in die Epoxyverbindung der Formel III und deren Isolierung erfolgt in an sich bekannter Weise, z.B. unter den aus der Steroidchemie, siehe C. Djerassi et al. "Steroid Reactions", Holden Day, San Francisco 1963, S. 606-613, bekannten Reaktionsbedingungen, insbesondere mittels einem der genannten Amidine, wie 1,5-Diazabicyclo[5.4.0]undec-5-en. Wenn die Verbindung der Formel III vor ihrer Weiterverarbeitung isoliert werden soll, benutzt man bevorzugt eines der genannten Amidine in etwa äquimolarer Menge.

Verbindungen der Formel V lassen sich beispielsweise herstellen, indem man eine Carbonsäure der Formel

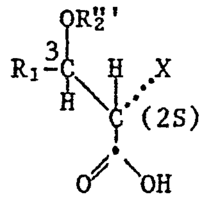

(VI)

worin $R_1$, $R_2'''$ und X die unter Formel V genannten Bedeutung haben, mit einem Amin der Formel

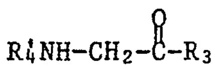

(VII),

worin $R_3$ und $R_4'$ die unter Formel V genannten Bedeutungen haben, umsetzt.

Die Umsetzung von Aminen der Formel VII mit einer Säure der Formel VI erfolgt auf an sich bekannte Weise unter acylierenden Bedingungen, beispielsweise, indem man die beiden Verbindungen in Gegenwart eines Kondensationsmittels miteinander kondensiert, oder ein reaktionsfähiges funktionelles Derivat einer Carbonsäure der Formel VI mit einem Amin der Formel VII umsetzt. Geeignete Kondensationsmittel sind beispielsweise Diimide, wie Diethyl- oder Dicyclohexylcarbodiimid, oder Di-N-heterocyclylcarbonylverbindungen, wie Carbonyldiimidazol, oder eine 1,2-Oxazoliumverbindung, wie 2-Ethyl-5-phenyl-1,2-oxazolium-3-sulfonat oder 2-tert-Butyl-5-methyl-1,2-oxazoliumperchlorat, die in Gegenwart eines inerten Lösungsmittels bei Raumtemperatur oder leicht erniedrigter oder erhöhter Temperatur angewendet werden. Reaktionsfähige funktionelle Derivate der Carbonsäure (VI) sind insbesondere Anhydride, insbesondere gemischte Anhydride, wie das entsprechende Carbonsäurechlorid oder -bromid, die gegebenenfalls in Gegenwart einer Base, mit dem Amin der Formel VII reagieren.

Verbindungen der Formel VI und VI', worin X Halogen ist, sind bekannt. Diese lassen sich beispielsweise in an sich bekannter Weise aus L-Serin oder L-Threonin bzw. deren D-Isomeren durch Diazotierung der Aminogruppe mit einem Nitritsalz, z.B. Kaliumnitrit, in Gegenwart einer Halogenwasserstoffsäure herstellen.

Bei Verwendung von L-Serin lassen sich nach den beschriebenen Verfahren ausgehend von Verbindungen der Formel VI Verbindungen der Formel I erhalten, worin der Kohlenstoff in 3-Stellung des Azetidinonrings die R-Konfiguration hat.

Bei Verwendung von L-Threonin erhält man Verbindungen der Formel I, worin das C-Atom in 3-Stellung des Azetidinonrings die R-Konfiguration und das 1'-C-Atom der Hydroxyethyl-Seitenkette die R-Konfiguration hat. Ist die Herstellung von Verbindungen der Formel I beabsichtigt, worin das 3-C-Atom die R-Konfiguration und das 1'-C-Atom der Hydroxyethyl-Seitenkette die S-Konfiguration hat, geht man von Allothreonin aus. Bei allen beschriebenen Umsetzungen bleibt die Konfiguration des C-Atoms, welches in Verbindungen der Formel I dem 1'-C-Atom der 3-Niederalkyl-CH-($OR_2$)-Seitenkette entspricht, unverändert.

Auf analoge Weise zur Herstellung von Verbindungen der Formel V lassen sich, ausgehend von Verbindungen der Formel VIII und den Verbindungen der Formel

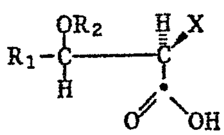

(VI')

die Verbindungen der Formel IV herstellen.

Verbindungen der Formel VII sind bekannt oder lassen sich, falls sie neu sind, in an sich bekannter Weise herstellen.

Die vorliegende Erfindung hat auch solche Ausführungsformen des Verfahrens zum Gegenstand, in denen man von einer auf irgendeiner Vorstufe des Verfahrens erhältlichen Verbindung ausgeht und die anschliessenden Verfahrensschritte ausführt.

Die Erfindung betrifft auch die Kombination der Verfahrensstufen ausgehend von den Verbindungen der Formeln VI, VI', VII und VIII bis zu den Verbindungen der Formel I.

Das erfindungsgemässe Verfahren hat den Vorteil, dass man die Verbindungen der Formel I in hoher Ausbeute und wirtschaftlicher Weise stereospezifisch herstellen kann und dass kristalline Zwischenprodukte erhalten werden.

Bevorzugt sind die neuen in den Beispielen angegebenen Verbindungen und Verfahren.

Die folgenden Beispiele dienen zur Illustration der Erfindung. Temperaturen sind in Graden Celsius und IR-Werte in $cm^{-1}$ angegeben. Für die chromato-graphische Trennung bei der präparativen Aufarbeitung lassen sich Säulen gefüllt mit Merck-Kieselgel 60 verwenden. Rf-Werte gelten für Fertigplatten für die Dünnschichtchromatographie (Merck 60 F 254).

Beispiel 1: (2R,3R)-N-Acetonyl-N-p-methoxyphenyl-2,3-epoxybuttersäureamid:

Zu einer auf -20° abgekühlten Lösung von 73,2 g N-Acetonyl-p-anisidin-hydrochlorid in 1700 ml Toluol werden unter Rühren (bei -15 bis -18°) nacheinander innerhalb 15 Minuten 70,2 g N,N-Dicyclohexylcarbodiimid, gelöst in 170 ml Toluol, und innerhalb 60 Minuten 103 g einer Lösung des Dicyclohexylammoniumsalzes der (2R,3R)-2,3-Epoxybuttersäure in 340 ml Dichlormethan zugetropft. Das Reaktionsgemisch lässt man innerhalb 45 Minuten auf 0° erwärmen und rührt weitere 18 1/2 Stunden bei 0-5°. Die Suspension wird abgenutscht, der Rückstand gründlich mit Toluol gewaschen, das Filtrat zweimal mit je 1 Liter Wasser gewaschen, getrocknet und im Wasserstrahlvakuum eingeengt. Aus dem rotbraunen, öligen Rohprodukt können nach Abkühlen auf ca. 4° Kristalle abgetrennt werden. Durch Flash-Chromatographie der Mutterlauge mit Hexan/Essigester-1:4-Gemisch wird, neben regeneriertem N-Acetonyl-p-anisidin, als Hauptprodukt in Form eines hellbraunen Oels das (2R,3R,)-N-Acetonyl-N-p-methoxyphenyl-2,3-epoxybuttersäureamid erhalten. Die Verbindung kann ohne weitere Reinigung direkt für die Zyklisation verwendet werden. IR: Banden u.a. bei 2840, 1735, 1670, 1510, 1240, 1220, 1170 und 840.

Beispiel 2: (3S,4S,1'R)-N-p-Methoxyphenyl-3-(1'-hydroxyethyl)-4-acetylazetidin-2-on:

Eine Lösung von 10,1 g (2R,3R)-N-Acetonyl-N-p-2,3-epoxybuttersäureamid in (200 ml) Dimethylformamid wird bei 60° mit 19.1 g Kaliumcarbonat versetzt und 13 Stunden bei gleicher Temperatur gerührt. Das abgekühlte Reaktionsgemisch wird von ungelöstem Kaliumcarbonat abfiltriert, mit 1 Liter Methylenchlorid versetzt und nacheinander fünfmal mit verdünnter, wässriger Natriumchlorid-Lösung gewaschen, getrocknet und eingedampft. Nach Flash-Chromatographie mit Hexan/-Ethylacetat (2:3) an 700 g Kieselgel wird als Hauptprodukt das nach Umkristallisieren aus Methylenchlorid/Ether bei 91-92° schmelzende (3S,4S,1'R)-N-pMethoxyphenyl-3-(1'-hydroxyethyl)-4-acetylazetidin-2-on ([α] = -119,9° (c = 0,853 in Chloroform) und als Nebenprodukt (ca. 10 %) das (3S,4R,1'R)-N-p-Methoxyphenyl-3-(1'-hydroxyethyl)-4-acetylazetidin-2-on (F. 165-168°) erhalten.

Beispiel 3: (3S,4S,1'R)-N-p-Methoxyphenyl-3-[1'-(dimethyl-2,3-dimethylbut-2-ylsilyloxy)ethyl]-4-acetylazetidin-2-on

Eine Lösung von 2,25 g (3S,4S,1'R)-N-p-Methoxyphenyl-3-(1'-hydroxyethyl)-4-acetylazetidin-2-on in 20 ml Dimethylformamid wird mit 0,85 g Imidazol und 1,7 ml Dimethyl-(2,3-dimethylbut-2-yl)-chlorsilan versetzt und 23 Stunden bei 25° gerührt. Nach Zugabe von weiteren 0,85 g Imidazol und 1,7 ml Dimethyl-(2,3-dimethylbut-2-yl)-chlorsilan wird weitere 20 Stunden gerührt. Das Reaktionsgemisch wird auf eiskalte, wässrige Natriumhydrogencarbonat-Lösung gegossen und mit Chloroform extrahiert. Die organische Phase wird nacheinander mit wässriger Natriumhydrogencarbonat-Lösung, 1 %-iger wässriger Citronensäure, wässriger Natriumhydrogencarbonat-Lösung und Wasser gewaschen, über Natriumsulfat getrocknet und im Vakuum eingedampft. Das anfallende Rohprodukt wird in Toluol/Ethylacetat (9:1) ans 280 g Kieselgel "Flash"-chromatographiert. Durch Vereinigen der einheitlichen Fraktionen erhält man das reine

(3S,4S,1'R)-N-p-Methoxyphenyl-3-[1'-(dimethyl-2,3-dimethylbut-2-ylsilyloxy)ethyl]-4-acetylazetidin-2-on. IR.: 2960, 2870, 1755, 1720, 1512, 1360, 1240, 1155, 1145, 830.

Beispiel 4: (3S,4S,1'R)-3-[1'-(Dimethyl-2,3-dimethylbut-2-yl-silyloxy)ethyl]-4-acetylazetidin-2-on:

Zu einer auf 0° abgekühlten Lösung von 3,5 g (3S,4S,1'R)-N-p-Methoxyphenyl-3-[1'-(dimethyl-2,3-dimethylbut-2-ylsilyloxy)ethyl]-4-acetylazetidin-2-on in 80 ml Acetonitril wird unter Argon innert 15 Minuten eine Lösung von 9,5 g Cer(IV)ammoniumnitrat in 45 ml Wasser zugetropft und das Reaktionsgemisch 1 Stunde bei 0-5° gerührt. Die organische Phase wird darauf abgetrennt und im Vakuum bei 30° eingedampft. Der Rückstand wird unter Zugabe von Eis mit der wässrigen Phase vereinigt und das Gemisch mit Essigester extrahiert. Der Extrakt wird nacheinander zweimal mit wässriger Natriumhydrogencarbonat-Lösung und verdünnter wässriger Natriumchlorid-Lösung gewaschen, getrocknet und eingedampft. Der Rückstand wird durch "Flash"-Chromatographie mit Hexan/Ethylacetat (80:20) an 300 g Kieselgel gereinigt. Die einheitliche Titelverbindung weist im IR.-Spektrum u.a. folgende Banden auf: 3410, 2960, 2870, 1775, 1725, 1140, 1105, 1075, 840.

Beispiel 5: (3R,4R,1'R)-3-[1'-(Dimethyl-2,3-dimethylbut-2-yl-silyloxy)ethyl]-4-acetoxyazetidin-2-on

Eine Lösung von 1,45 g (3S,4S,1'R)-3-[1'-(Dimethyl-2,3-dimethyl-but-2-ylsilyloxy)ethyl]-4-acetylazetidin-2-on in 75 ml Methylenchlorid wird mit 5,8 g 85%iger m-Chlorperbenzoesäure versetzt und 3,5 Stunden bei Raumtemperatur belassen. Das Reaktionsgemisch giesst man darauf auf Eiswasser, extrahiert mit Methylenchlorid nach wäscht die organische Phase nacheinander mit wässriger Kaliumjodid/Natriumthiosulfat-Lösung, Wasser, wässriger Natriumhydrogencarbonat-Lösung und Wasser, trocknet und dampft ein. Beim Versetzen des Rohproduktes mit Ether kristallisieren kleine Mengen 3-Chlorbenzoylperoxid aus. Die Kristalle werden abgenutscht und das Filtrat durch "Flash"-chromatographie Ethylacetat (90:10) gereinigt. Die einheitlichen Fraktionen liefern nach Umlösen aus

Hexan das reine, bei 79-80°C schmelzende (3R,4R,1'R)-3-[1'-(Dimethyl-2,3-dimethylbut-2-ylsilyloxy)ethyl]-4-acetoxyazetidin-2-on. IR.-Spektrum: 3410, 2960, 2870, 1790, 1745, 1235, 1180, 1035, 835. DC: Silicagel (System Toluol/Aethylacetat 3:1): $R_f$ = 0,45.

Beispiel 6: (3S,4R,1'R)-3-[1'-(Dimethyl-2,3-dimethylbut-2-ylsilyloxy)ethyl]-4-N-allyloxycarbonylglycylthioazetidin-2-on

1,35 g N-Allyloxycarbonylthioglycin-Dicyclohexylammoniumsalz werden in 3,8 ml N NaOH und 20 ml Wasser gelöst. Die entstandene Lösung wird dreimal mit Methylenchlorid extrahiert, der wässrige Anteil mit 5 ml Aceton und bei 0° unter Rühren unter Argon mit einer Lösung von 410 mg (3R,4R,1'R)-3-[1'-(Dimethyl-2,3-dimethylbut-2-ylsilyloxy)-ethyl]4-acetoxyazetidin-2-on in 20 ml Aceton versetzt. Das Reaktionsgemisch wird bei einem pH von 10,5-10,7 während 5 Stunden unter Kühlen weitergerührt. Das Aceton wird unter vermindertem Druck abdestilliert, der wässrige Rückstand mit Methylenchlorid extrahiert, die organische Phase nach Trocknen über $Na_2SO_4$ eingedampft und der Rückstand an Kieselgelplatten in Hexan/Ethylacetat (1:1) chromatographiert. Man erhält die Titelverbindung als farblose, kristalline Verbindung, welche aus Ether-Hexan umkristallisiert wird. F. 74-76°.
$[\alpha]$ = + 110,7 ± 1,3° (c = 0,76 % in $CHCl_3$).

Beispiel 7: (5R,6S,1'R)-6-[1'-(Dimethyl-2,3-dimethylbut-2-ylsilyloxy)ethyl]-2-allyloxycarbonylaminomethyl-2-penem-3-carbonsäureallylester

Eine Lösung von 692 mg (1,6 mMol) (3S,4R,1'R)-3-[1'-(Dimethyl-2,3-dimethylbut-2-ylsilyloxy)ethyl]-4-N-allyloxycarbonylglycylthioazetidin-2-on in 9 ml Methylenchlorid wird bei -20° unter Argon nacheinander mit 360 mg (ca. 2,41 mMol) Oxalsäureallylesterchlorid und 410 ml (ca. 2,41 mMol) Hünig-Base versetzt und das entstandene Reaktionsgemisch zuerst 2,5 Stunden bei -10° und anschliessend 80 Minuten bei 0° gerührt. Es wird mit Methylenchlorid verdünnt und nacheinander mit eiskalter 0,1N HCl- und 8 %iger wässriger $NaHCO_3$-Lösung gewaschen. Der organische Anteil wird über $Na_2SO_4$ getrocknet und eingedampft. Der erhaltene Rückstand wird in 3 ml Triethylphosphit gelöst und die Lösung 2 Stunden unter Argon auf 60° erwärmt. Die flüchtigen Anteile werden zunächst unter vermindertem Druck, dann im Hochvakuum entfernt, der Rückstand in 10 ml abs. Dioxan gelöst und die resultierende Lösung unter Argon 8 Stunden auf 105° (Badtemperatur) erwärmt. Das nach Eindampfen von Dioxan unter vermindertem Druck erhaltene Rohprodukt wird auf einer Kieselgelsäule mit Hexan/Ethylacetat (9:1 und 4:1) chromatographiert. Die Titelverbindung wird als farbloses Oel erhalten. [α] = +62 ± 2,7° (c = 0,371 % in $CHCl_3$), U.V.: $\lambda_{max}$=320 nm (ε=6230; in Ethanol).

Beispiel 8: (5R,6S,1'R)-2-Aminomethyl-6-[1'-(dimethyl-2,3-dimethylbut-2-ylsilyloxy)ethyl]-2-penem-3-carbonsäure

Eine Lösung von 367 mg (0,72 mMol) (5R,6S,1'R)-6-[1'-Dimethyl-(2,3-dimethylbut-2-ylsilyloxy)ethyl]-2-allyloxycarbonylaminomethyl-2-penem-3-carbonsäureallylester in 7 ml abs. Tetrahydrofuran wird bei Raumtemperatur mit 223 mg (1,59 mMol) Dimedon und 36 mg Tetrakis-triphenylphosphin-palladium versetzt und die entstandene Lösung bei Raumtemperatur weitergerührt. Bald beginnt die Ausscheidung der kristallinen Titelverbindung und ist nach 1 Stunde praktisch beendet. Die Kristalle werden abfiltriert und auf dem Filter mit etwas Tetrahydrofuran nachgewaschen. Die erhaltene Titelverbindung bildet farblose Kristalle, F. 119°;
UV: $\lambda_{max}$ (EtOH):309 nm, 268 nm.

Beispiel 9: (5R,6S,1'R)-6-(1'-Hydroxyethyl)-2-allyloxycarbonylaminomethyl-2-penem-3-carbonsäureallylester

Eine Lösung von 50 mg (5R,6S,1'R)-6-[1'-(Dimethyl-2,3-dimethylbut-2-ylsilyloxy)ethyl]-2-allyloxycarbonylaminomethyl-2-penem-3-carbonsäureallylester in 1 ml 2,6-Lutidin wird mit 60 mg (ca. 30mMol) einer 70%igen HF-Harnstoff-Mischung versetzt und 2 Stunden bei Raumtemperatur gerührt. Dann werden zur Reaktionslösung weitere 5 Tropfen (ca. 100 mg = ca. 50 mM) HF-Harnstoff zugefügt. Das viskose Gemisch wird mit 1 ml Methylenchlorid verdünnt, über Nacht bei Raumtemperatur gerührt und erneut mit 10 Tropfen HF-Harnstoff (ca. 100 mM) versetzt. Nach weiteren 24 Stunden Reaktionszeit bei Raumtemperatur wird das Reaktionsgemisch mit Wasser verdünnt und mit

Essigester extrahiert. Die organische Phase wird anschliessend mit 4N Salzsäure und mit gesättigter, wässriger Natriumchlorid-Lösung gewaschen, getrocknet und im Rotationsverdampfer eingeengt. Durch Umlösen des anfallenden Rohproduktes aus Ether/Hexan wird die reine Titelverbindung gewonnen. F. 141-141,5°. ($R_f$ Merck Fertigplatten, Toluol/Essigester 1:1) : 0,20.

Beispiel 10: (5R,6S,1'R)-2-Aminomethyl-6-(1'-hydroxyethyl)-2-penem-3-carbonsäure

Eine Lösung von 313 mg (0,85 mmol) (5R,6S,1'R)-2-Allyloxycarbonyl-aminomethyl-6-(1'-hydroxyethyl)-2-penem-3-carbonsäureallylester und 174,6 mg (1,24 mmol) Dimedon in 7,5 ml Tetrahydrofuran wird während 5 Minuten mit Argon gespült und unter Argon-Atmosphäre bei Raumtemperatur mit 27 mg (0,023 mmol) Tetrakistriphenylphosphinpalladium versetzt. Nach ca. 5 Min beginnt ein Niederschlag auszufallen, der nach weiteren 2 Stunden Rühren abfiltriert, mit ca. 10 ml Tetrahydrofuran gewaschen und anschliessend in 4 ml $H_2O$ gelöst wird. Zur beigen Lösung wird eine Mikrospatelspitze Aktivkohle und 2 Mikrospatelspitzen Tonsil gegeben, 5 Min gut gerührt und über Hyflo klar filtriert. Am Hochvakuum wird eingeengt und der erhaltene dicke Kristallbrei mit 3 ml kaltem Ethanol versetzt und abgenutscht. Die farblosen Kristalle werden mit 2 ml Tetrahydrofuran gewaschen und am Hochvakuum getrocknet. Man erhält die Titelverbindung vom Rf-Wert = 0,50 (DC:$H_2O$, OPTI $UPC_{12}$); $[\alpha]_D^{20}$ = +176° (c = 0,5, $H_2O$).

Beispiel 11: (1'R,3R,4R)-3-(1'Hydroxyethyl)-4-acetoxyazetidin-2-on

Eine Lösung von 567 mg (1,8 mmol) (3R,4R,1'R)-3-[1'-(Dimethyl-2,3-dimethylbut-2-ylsilyloxy)ethyl]-4-acetoxyazetidin-2-on und 504 mg (14,4 mmol) 57 % HF-Harnstoff in 5,5 ml Acetonitril wird über Nacht (total 17 Stunden) bei Raumtemperatur gerührt. Nach portionenweiser Zugabe von 2,42 g Natriumbicarbonat und einstündigem Rühren bei Raumtemperatur wird filtriert und die Lösung eingedampft. Der Rückstand wird mit Essigester chromatographiert und ergibt die Titelverbindung vom Schmelzpunkt 110-112° (Essigester).

Die erhaltene Titelverbindung kann auf an sich bekannte Weise, insbesondere aber gemäss der Schweizer Patentanmeldung Nr. 919/86-5 in Penemverbindungen übergeführt werden.

Patentansprüche für die Vertragsstaaten: DE, GB, FR, CH, LI, IT, NL, BE, SE, LU

1. Optisch aktive Acetyloxyazetidinone der Formel

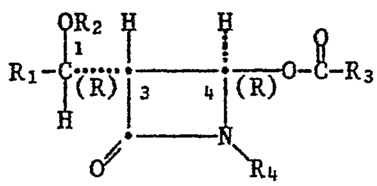

(I) ,

worin $R_1$ Wasserstoff oder Niederalkyl, $R_2$ Dimethyl-(2,3-dimethylbut-2-yl)silyl $R_3$ Methyl, $R_4$ Wasserstoff oder eine Aminoschutzgruppe $R_4'$ bedeutet.

2. Verbindungen der Formel I nach Anspruch 1, worin $R_1$ Methyl ist.

3. Verbindungen der Formel I nach Anspruch 1, worin $R_4$ Wasserstoff oder p-Methoxyphenyl ist.

4. (3R,4R,1'R)-3-[1'-(Dimethyl-2,3-dimethylbut-2-ylsilyloxy)-ethyl]-4-acetyloxyazetidin-2-on nach Anspruch 1.

5. Verfahren zur Herstellung von optisch aktiven Acetyloxyazetidinonen der Formel

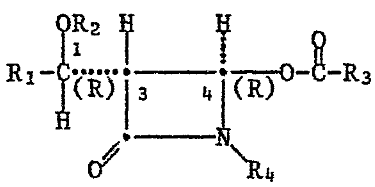

(I) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeuten, dadurch gekennzeichnet, dass man eine Verbindung der Formel

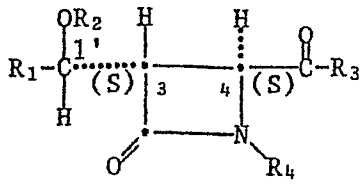

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, oder in einer Verbindung der Formel I, worin $R_2$ Wasserstoff bedeutet, die freie Hydroxygruppe in die geschützte Hydroxygruppe $OR_2$ überführt, worin $R_2$ Dimethyl-(2,3-dimethylbut-2-yl)silyl ist, und/oder gewünschtenfalls in einer erhältlichen Verbindung der Formel I die Aminoschutzgruppe $R_4'$ abspaltet.

6. Eine Verbindung der Formel

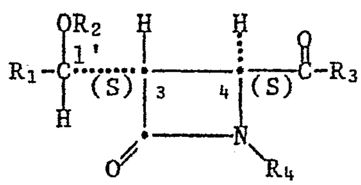

(II) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeutet.

7. Eine Verbindung der Formel II nach Anspruch 6, worin $R_1$ Methyl und $R_4$ Wasserstoff oder eine Aminoschutzgruppe $R_4'$ bedeuten.

8. (3S,4S,1'R)-3-[1'-(Dimethyl-2,3-dimethylbut-2-ylsilyloxy)-ethyl]-4-acetylazetidin-2-on nach Anspruch 6.

9. Verfahren zur Herstellung von Verbindungen der Formel

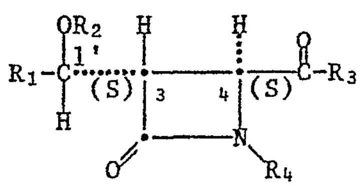

(II) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeutet, dadurch gekennzeichnet, dass man a) ein α-Carbanion einer Verbindung der Formel

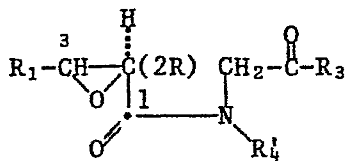
(III) ,

worin $R_1$, $R_3$ und $R_4'$ die unter Formel II genannten Bedeutungen haben, und das 2-C-Atom die R- und, wenn $R_1$ Niederalkyl bedeutet, das 3-C-Atom die R- oder die S-Konfiguration hat, oder eine Verbindung der Formel

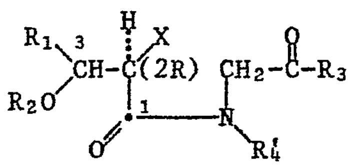
(IV),

worin $R_1$, $R_2$, $R_3$ und $R_4'$ die unter Formel II genannten Bedeutungen haben, und X eine nukleofuge Abgangsgruppe darstellt und das 2-C-Atom die R- und, wenn $R_1$ Niederalkyl bedeutet, das 3-C-Atom die R- oder die S-Konfiguration hat, zyklisiert oder b) eine cis-Verbindung der Formel

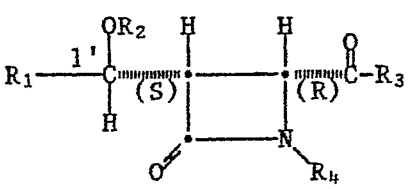
(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder eine erhältliche Verbindung der Formel II, worin $R_2$ Wasserstoff ist mit einem Dimethyl-(2,3-dimethylbut-2-yl)halogensilan umsetzt.

10. Verfahren nach Anspruch 9 zur Herstellung von Verbindungen der Formel II, worin $R_1$ Methyl und $R_4$ Wasserstoff oder p-Methoxyphenyl bedeuten.

11. Verfahren zur Herstellung von (3S,4S,1'R)-N-p-Methoxyphenyl-3-[1'-(dimethyl-2,3-dimethylbut-2-ylsilyloxy]ethyl]-4-acetylazetidin-2-on nach Anspruch 10.

12. Verfahren zur Herstellung von (3S,4S,1'R)-3-[1'-(Dimethyl-2,3-dimethylbut-2-ylsilyloxy)ethyl]-4-acetylazetidin-2-on nach Anspruch 10.

13. Verfahren zur Herstellung von (4R)-3,4-trans-disubstituierten Azetidinonen der Formel

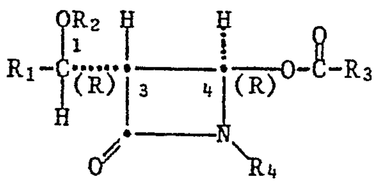

(I) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeuten, dadurch gekennzeichnet, dass man eine Verbindung der Formel

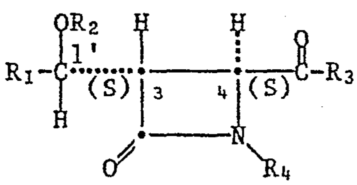

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, oder in einer Verbindung der Formel I, worin $R_2$ Wasserstoff bedeutet, die freie Hydroxygruppe in die geschützte Hydroxygruppe $OR_2$ überführt, worin $R_2$ Dimethyl-(2,3-dimethylbut-2-yl)silyl ist, und/oder gewünschtenfalls in einer erhältlichen Verbindung der Formel I die Aminoschutzgruppe $R_4'$ abspaltet, wobei das Verfahren zur Herstellung von Verbindungen der Formel

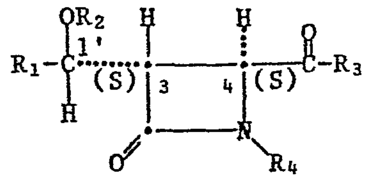

(II) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeutet, dadurch gekennzeichnet ist, dass man a) ein α-Carbanion einer Verbindung der Formel

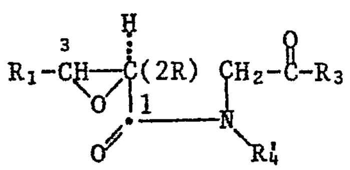

(III) ,

worin $R_1$, $R_3$ und $R_4'$ die unter Formel II genannten Bedeutungen haben, und das 2-C-Atom die R- und, wenn $R_1$ Niederalkyl bedeutet, das 3-C-Atom die R- oder die S-Konfiguration hat, oder eine Verbindung der Formel

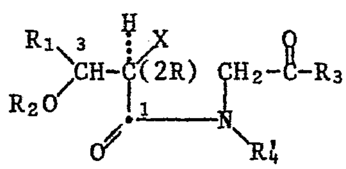

(IV),

worin $R_1$, $R_2$, $R_3$ und $R_4'$ die unter Formel II genannten Bedeutungen haben, und X eine nukleofuge Abgangsgruppe darstellt und das 2-C-Atom die R- und, wenn $R_1$ Niederalkyl bedeutet, das 3-C-Atom die R- oder die S-Konfiguration hat, zyklisiert oder b) eine cis-Verbindung der Formel

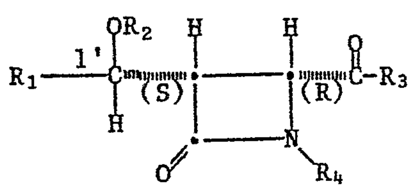

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder eine erhältliche

Verbindung der Formel II, worin $R_2$ Wasserstoff ist, mit einem Dimethyl-(2,3-dimethylbut-2-yl)halogensilan umsetzt, das Verfahren zur Herstellung von Verbindungen der Formel

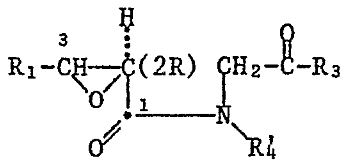

(III) ,

worin $R_1$ Wasserstoff oder Niederalkyl, $R_3$ Methyl und $R_4'$ eine Aminoschutzgruppe bedeutet, und das 2-C-Atom die R- und, wenn $R_1$ Niederalkyl bedeutet, das 3-C-Atom die R- oder die S-Konfiguration hat, dadurch gekennzeichnet ist, dass man eine Verbindung der Formel

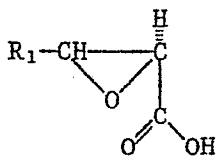

(VIII)

mit einem Amin der Formel

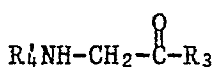

(VII),

worin $R_3$ und $R'_4$ die unter Formel (V) genannten Bedeutungen haben, umsetzt oder aus Verbindungen der Formel

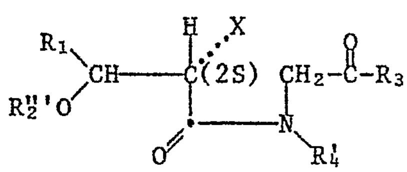

(V),

worin $R_1$, $R_3$, $R_4'$ und X die genannten Bedeutungen haben, $R_2'''$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ Niederalkyl bedeutet, unter carbonionenbildenden Bedingungen und unter Walderscher Umkehr am 2-C-Atom in situ herstellt,

das Verfahren zur Herstellung von Verbindungen der Formel

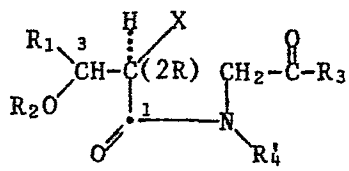

(IV),

worin $R_1$ Wasserstoff oder Niederalkyl, $R_2$ Dimethyl-(2,3-dimethylbut-2-yl)silyl, $R_3$ Methyl, $R'_4$ eine Aminoschutzgruppe, und X eine nukleofuge Abgangsgruppe darstellt und das 2-C-Atom die R- und, wenn $R_1$ Niederalkyl bedeutet, das 3-C-Atom die R- oder die S-Konfiguration hat, dadurch gekennzeichnet ist, dass man eine Carbonsäure der Formel

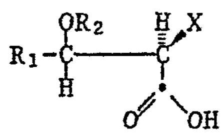

(VI')

worin $R_1$, $R_2$ und X die unter Formel IV genannten Bedeutungen haben, mit einem Amin der Formel

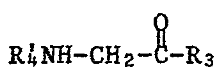

(VII),

worin $R_3$ und $R'_4$ die unter Formel (V) genannten Bedeutungen haben, umsetzt und
das Verfahren zur Herstellung von Verbindungen der Formel

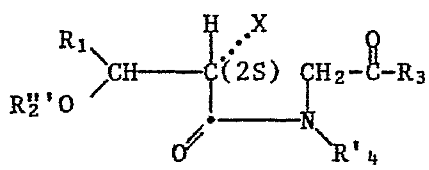

(V),

worin $R_1$ Wasserstoff oder Niederalkyl, $R'''_2$ Wasserstoff oder eine unter den Bedingungen der Epoxidbildung abspaltbare Hydroxyschutzgruppe, $R_3$ Methyl, $R_4$ eine Aminoschutzgruppe und X eine nukleofuge Abgansgruppe darstellen und worin das 3-C-Atom die R-Konfiguration oder die 3S-Konfiguration hat, wenn $R_1$ Niederalkyl bedeutet, dadurch gekennzeichnet ist, dass man eine Carbonsäure der Formel

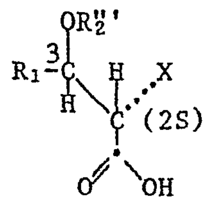

(VI)

worin $R_1$ $R'''_2$ und X die unter Formel (V) genannten Bedeutung haben, mit einem Amin der Formel

$$R'_4NH-CH_2-\overset{O}{\overset{\|}{C}}-R_3 \qquad (VII),$$

worin $R_3$ und $R'_4$ die unter Formel (V) genannten Bedeutungen haben, umsetzt.

14. Verfahren nach Anspruch 5 oder 13, dadurch gekennzeichnet, dass man von Verbindungen der Formel II ausgeht, worin $R_1$ Methyl ist.

15. Verfahren nach Anspruch 5 oder 13, dadurch gekennzeichnet, dass man von Verbindungen der Formel II ausgeht, worin $R_4$ Wasserstoff oder p-Methoxyphenyl ist.

16. Verfahren zur Herstellung von (3R,4R,1'R)-3-[1'-(Dimethyl-2,3-dimethylbut-2-ylsilyloxy)ethyl]-4-acetyloxyazetidin-2-on nach Anspruch 5 oder 13.

FO 7.4/WAR/sm*/cc*

Patentansprüche: (für die Vertragsstaaten AT und ES)

1. Verfahren zur Herstellung von optisch aktiven Acetyloxyazetidinonen der Formel

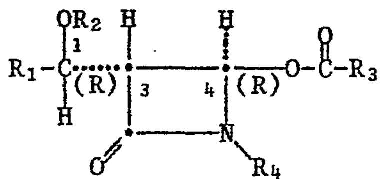

(I) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeuten, dadurch gekennzeichnet, dass man eine Verbindung der Formel

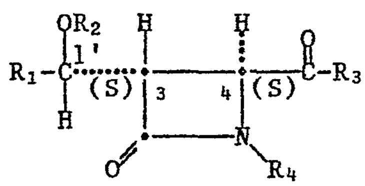

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, oder in einer Verbindung der Formel I, worin $R_2$ Wasserstoff bedeutet, die freie Hydroxygruppe in die geschützte Hydroxygruppe $OR_2$ überführt, worin $R_2$ Dimethyl-(2,3-dimethylbut-2-yl)silyl ist, und/oder gewünschtenfalls in einer erhältlichen Verbindung der Formel I die Aminoschutzgruppe $R_4'$ abspaltet.

2. Verfahren zur Herstellung von Verbindungen der Formel

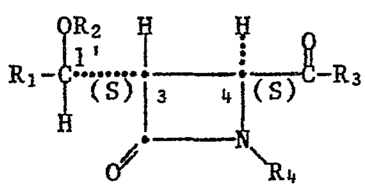

(II) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeutet, dadurch gekennzeichnet, dass man a) ein α-Carbanion einer Verbindung der Formel

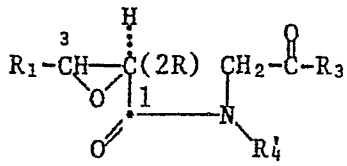

(III) ,

worin $R_1$, $R_3$ und $R_4'$ die unter Formel II genannten Bedeutungen haben, und das 2-C-Atom die R- und, wenn $R_1$ Niederalkyl bedeutet, das 3-C-Atom die R- oder die S-Konfiguration hat, oder eine Verbindung der Formel

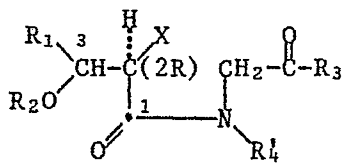

(IV),

worin $R_1$, $R_2$, $R_3$ und $R_4'$ die unter Formel II genannten Bedeutungen haben, und X eine nukleofuge Abgangsgruppe darstellt und das 2-C-Atom die R- und, wenn $R_1$ Niederalkyl bedeutet, das 3-C-Atom die R- oder die S-Konfiguration hat, zyklisiert oder b) eine cis-Verbindung der Formel

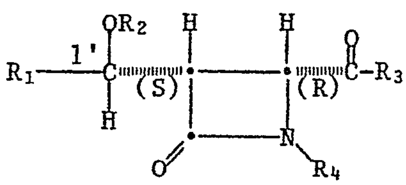

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder eine erhältliche Verbindung der Formel II, worin $R_2$ Wasserstoff ist mit einem Dimethyl-(2,3-dimethylbut-2-yl)halogensilan umsetzt.

3. Verfahren nach Anspruch 9 zur Herstellung von Verbindungen der Formel II, worin $R_1$ Methyl und $R_4$ Wasserstoff oder p-Methoxyphenyl bedeuten.

4. Verfahren zur Herstellung von (3S,4S,1'R)-N-p-Methoxyphenyl-3-[1'-(dimethyl-2,3-dimethylbut-2-ylsilyloxy]ethyl]-4-acetylazetidin-2-on nach Anspruch 2.

5. Verfahren zur Herstellung von (3S,4S,1'R)-3-[1'-(Dimethyl-2,3-dimethylbut-2-ylsilyloxy)ethyl]-4-acetylazetidin-2-on nach Anspruch 2.

6. Verfahren zur Herstellung von (4R)-3,4-trans-disubstituierten Azetidinonen der Formel

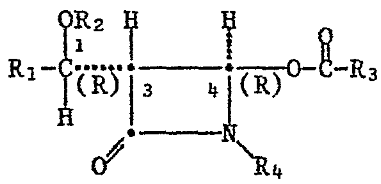

(I) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeuten, dadurch gekennzeichnet, dass man eine Verbindung der Formel

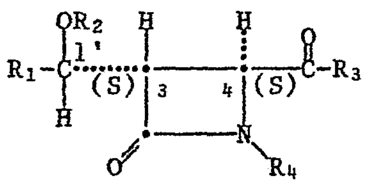

(II) ,

worin $R_1$, $R_2$, $R_3$ und $R_4$ die unter Formel I genannten Bedeutungen haben, durch Behandeln mit einer Persäure einer Bayer-Villiger-Reaktion unterwirft, oder in einer Verbindung der Formel I, worin $R_2$ Wasserstoff bedeutet, die freie Hydroxygruppe in die geschützte Hydroxygruppe $OR_2$ überführt, worin $R_2$ Dimethyl-(2,3-dimethylbut-2-yl)silyl ist, und/oder gewünschtenfalls in einer erhältlichen Verbindung der Formel I die Aminoschutzgruppe $R_4'$ abspaltet, wobei das Verfahren zur Herstellung von Verbindungen der Formel

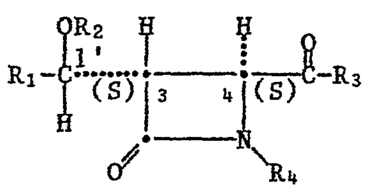

(II) ,

worin $R_1$ Wasserstoff oder Niederalkyl, $R_2$ Dimethyl-(2,3-dimethylbut-2-yl)silyl, $R_3$ Methyl und $R_4$ Wasserstoff oder eine Aminoschutzgruppe $R_4'$ bedeutet, dadurch gekennzeichnet ist, dass man a) ein α-Carbanion einer Verbindung der Formel

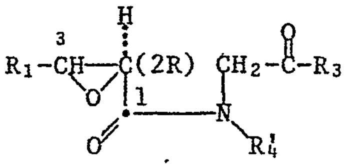

(III) ,

worin $R_1$, $R_3$ und $R_4'$ die unter Formel II genannten Bedeutungen haben, und das 2-C-Atom die R- und, wenn $R_1$ Niederalkyl bedeutet, das 3-C-Atom die R- oder die S-Konfiguration hat, oder eine Verbindung der Formel

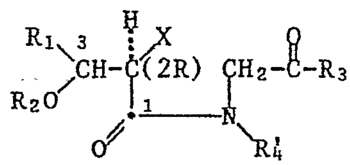

(IV),

worin $R_1$, $R_2$, $R_3$ und $R_4'$ die unter Formel II genannten Bedeutungen haben, und X eine nukleofuge Abgangsgruppe darstellt und das 2-C-Atom die R- und, wenn $R_1$ Niederalkyl bedeutet, das 3-C-Atom die R- oder die S-Konfiguration hat, zyklisiert oder b) eine cis-Verbindung der Formel

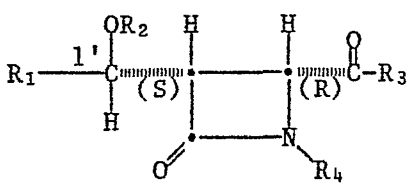

(II') ,

worin $R_1$, $R_2$, $R_3$ und $R_4$ die unter Formel II angegebenen Bedeutungen haben, isomerisiert, und, wenn erwünscht, die Aminoschutzgruppe $R_4'$ abspaltet und durch Wasserstoff ersetzt und/oder eine erhältliche Verbindung der Formel II, worin $R_2$ Wasserstoff ist, mit einem Dimethyl-(2,3-dimethylbut-2-yl)halogensilan umsetzt, das Verfahren zur Herstellung von Verbindungen der Formel

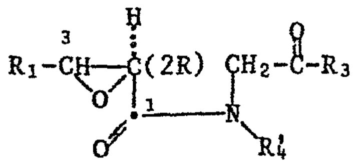

(III) ,

worin $R_1$ Wasserstoff oder Niederalkyl, $R_3$ Methyl und $R_4'$ eine Aminoschutzgruppe bedeutet, und das 2-C-Atom die R- und, wenn $R_1$ Niederalkyl bedeutet, das 3-C-Atom die R- oder die S-Konfiguration hat, dadurch gekennzeichnet ist, dass man eine Verbindung der Formel

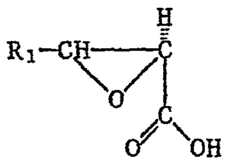

(VIII)

mit einem Amin der Formel

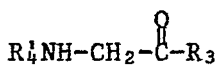

(VII),

worin $R_3$ und $R'_4$ die unter Formel (V) genannten Bedeutungen haben, umsetzt oder aus Verbindungen der Formel

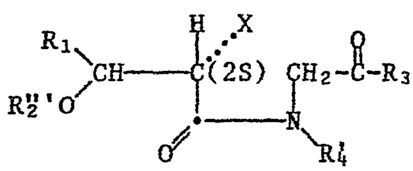

(V),

worin $R_1$, $R_3$, $R_4'$ und X die genannten Bedeutungen haben, $R_2''{}'$ Wasserstoff oder eine unter den Bedingungen der Epoxidbildung abspaltbare Hydroxyschutzgruppe darstellt und worin das 3-C-Atom die R- oder S-Konfiguration hat, wenn $R_1$ Niederalkyl bedeutet, unter carbonionenbildenden Bedingungen und unter Walderscher Umkehr am 2-C-Atom <u>in situ</u> herstellt,

das Verfahren zur Herstellung von Verbindungen der Formel

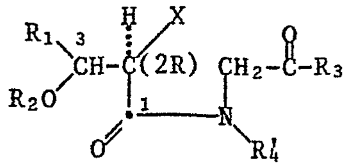

(IV),

worin $R_1$ Wasserstoff oder Niederalkyl, $R_2$ Dimethyl-(2,3-dimethylbut-2-yl)silyl, $R_3$ Methyl, $R_4'$ eine Aminoschutzgruppe, und X eine nukleofuge Abgangsgruppe darstellt und das 2-C-Atom die R- und, wenn $R_1$ Niederalkyl bedeutet, das 3-C-Atom die R- oder die S-Konfiguration hat, dadurch gekennzeichnet ist, dass man eine Carbonsäure der Formel

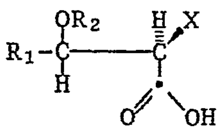

(VI')

worin $R_1$, $R_2$ und X die unter Formel IV genannten Bedeutungen haben, mit einem Amin der Formel

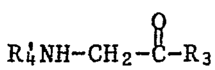

(VII),

worin $R_3$ und $R'_4$ die unter Formel (V) genannten Bedeutungen haben, umsetzt und
das Verfahren zur Herstellung von Verbindungen der Formel

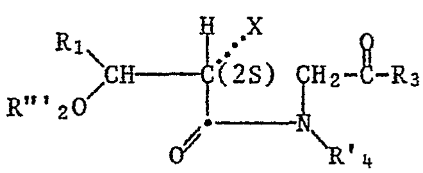

(V),

worin $R_1$ Wasserstoff oder Niederalkyl, $R'''_2$ Wasserstoff oder eine unter den Bedingungen der Epoxidbildung abspaltbare Hydroxyschutzgruppe, $R_3$ Methyl, $R_4$ eine Aminoschutzgruppe und X eine nukleofuge Abgangsgruppe darstellen und worin das 3-C-Atom die R-Konfiguration oder die S-Konfiguration hat, wenn $R_1$ Niederalkyl bedeutet, dadurch gekennzeichnet ist, dass man eine Carbonsäure der Formel

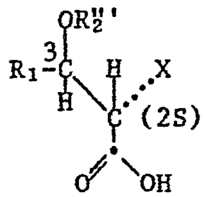

(VI)

worin $R_1$ $R'''_2$ und X die unter Formel (V) genannten Bedeutung haben, mit einem Amin der Formel

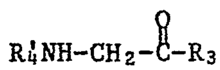

(VII),

worin $R_3$ und $R'_4$ die unter Formel (V) genannten Bedeutungen haben, umsetzt.

7. Verfahren nach Anspruch 1 oder 6, dadurch gekennzeichnet, dass man von Verbindungen der Formel II ausgeht, worin $R_1$ Methyl ist.

8. Verfahren nach Anspruch 1 oder 6, dadurch gekennzeichnet, dass man von Verbindungen der Formel II ausgeht, worin $R_4$ Wasserstoff oder p-Methoxyphenyl ist.

9. Verfahren zur Herstellung von (3R,4R,1'R)-3-[1'-(Dimethyl-2,3-dimethylbut-2-ylsilyloxy)ethyl]-4-acetyloxyazetidin-2-on nach Anspruch 1 oder 6.

FO 7.4/WAR/sm*/cc*

0221846

Europäisches Patentamt

# EUROPÄISCHER RECHERCHENBERICHT

Nummer der Anmeldung

EP 86 81 0474

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | CHEMICAL ABSTRACTS, Band 103, Nr. 15, 14. Oktober 1985, Seite 706, Zusammenfassung Nr. 123272t, Columbus, Ohio, US; & JP-A-60 61 566 (SANKYO CO., LTD) 09-04-1985<br>* Zusammenfassung *<br>--- | 1,5,6,9 | C 07 F 7/18 //<br>C 07 D 205/08<br>C 07 D 499/00 |
| Y | TETRAHEDRON LETTERS, Band 26, Nr. 45, 1985, Seiten 5515-5518, Pergamon Press; H. WETTER et al.: "Thexyldimethylsilyl chloride, an easily accessible reagent for the protection of alcohols"<br>* Insgesamt *<br>--- | 1 | |
| | | | RECHERCHIERTE SACHGEBIETE (Int Cl 4) |
| A | TETRAHEDRON, Band 39, Nr. 14, Seiten 2399-2407, Pergamon Ltd, GB; M. SHIOZAKI: "Stereocontrolled syntheses of chiral and racemic key intermediates to thienamycin from d-allo-threonine and trans-crotonic acid"<br>* Seite 2401; Seite 2404, Spalte 2 *<br>--- | 1,5 | C 07 F 7/00<br>C 07 D 205/00<br>C 07 D 499/00 |
| P,Y | EP-A-0 181 831 (CIBA-GEIGY)<br>* Seiten 33,34, Beispiele 22-24; Ansprüche *<br>----- | 1-3,5-7,9,10,13-15 | |

Der vorliegende Recherchenbericht wurde für alle Patentansprüche erstellt.

| Recherchenort | Abschlußdatum der Recherche | Prüfer |
|---|---|---|
| DEN HAAG | 22-01-1987 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82